(19)

Europäisches
Patentamt

European
Patent Office

Office européen
des brevets

(11) **EP 1 404 744 B1**

(12) **EUROPEAN PATENT SPECIFICATION**

(45) Date of publication and mention
of the grant of the patent:
**07.10.2009 Bulletin 2009/41**

(21) Application number: **01959752.5**

(22) Date of filing: **13.08.2001**

(51) Int Cl.:
**C08G 77/50** $^{(2006.01)}$     **C08L 83/14** $^{(2006.01)}$
**C08L 83/04** $^{(2006.01)}$     **A61K 8/89** $^{(2006.01)}$

(86) International application number:
**PCT/US2001/025569**

(87) International publication number:
**WO 2002/100922 (19.12.2002 Gazette 2002/51)**

(54) **BRANCH ORGANOSILICONE COMPOUND**

VERZWEIGTE ORGANOSILIKONVERBINDUNG

COMPOSE ORGANOSILICIE RAMIFIE

(84) Designated Contracting States:
**DE FR GB NL**

(30) Priority: **11.06.2001   US 878888**

(43) Date of publication of application:
**07.04.2004   Bulletin 2004/15**

(73) Proprietor: **Momentive Performance Materials Inc.
Albany, New York 12211 (US)**

(72) Inventors:
• **KILGOUR, John, Alfred
Clifton Park, NY 12065 (US)**

• **CHAIYAWAT, Atchara
Williamstown WV 26187 (US)**
• **LAI, Kuo-Tsai Griffin
Rincon GA 31326 (US)**

(74) Representative: **Herrmann, Uwe et al
Lorenz - Seidler - Gossel
Widenmayerstrasse 23
80538 München (DE)**

(56) References cited:
**EP-A- 0 422 805         EP-A- 0 694 573
EP-A- 0 922 734         EP-A- 0 958 804
EP-A- 1 002 825         EP-A- 1 164 171
EP-A- 1 164 172         WO-A-01/14458**

EP 1 404 744 B1

**Description**

CROSS-REFERENCE TO RELATED APPLICATIONS

This application claims rights of priority from U.S. Provisional Patent Application Serial No. 60/211,306, filed June 13, 2000.

FIELD OF THE INVENTION

The invention relates to silicone materials, more specifically, to organosiloxane compounds that exhibit branching.

BRIEF DESCRIPTION OF THE RELATED ART

The personal care industry thrives on being able to deliver multiple performance products based on mixture of several components, with each having performance characteristics important to the final formulation. One important characteristic is the ability to provide a silky initial feel derived from low molecular weight silicones, such as for example, octamethylcyclotetrasilioxane or decamethylcyclopentasiloxane, in the formulation while maintaining a high but shear-thinnable viscosity. While these low molecular weight silicones provide the desired feel characteristics, they are also low viscosity, highly flowable liquids. Thus they are not easily held in a formulation, preferring rather to separate and flow out of a given container or flow uncontrollably when used in a specific application. Further, it desirable to achieve the initial silky feel while providing a smooth, low-residue sensory feel on dry-down. U.S. Patent Nos. 5,493,041 and 4,987,169 and coassigned U.S. Patent No. 5,760,116 each disclose the use of polymeric silicone gels prepared in volatile silicone oils to deliver the desirable initial feel of volatile, low viscosity silicones to formulations while at the same time provide high viscosity and a smooth silky feel on dry-down. EP-A-1 164 171, EP-A-0 958 804, EP-A-0 694 573 and EP-A-0 827 983 disclose branched organosiloxane silicone compositions according to the preamble part of claim 1. EP-A-1 164 172 discloses a similar composition.

SUMMARY OF THE INVENTION

In a first aspect, the present invention relates to a branched organosiloxane compound, comprising, per molecule of the compound, a silicone core and one or more hydrocarbon terminated branches attached to the silicone core.
In a second aspect, the present invention relates to a method for making a branched organosiloxane compound comprising contacting under hydrosilylation conditions, a silylhydride functional organosiloxane, a monoethylenically unsaturated hydrocarbon and a polyethylenically unsaturated siloxane resin
In a third aspect, the present invention relates to a silicone composition, comprising:

(a) a network comprising two or more molecules of a branched organosiloxane compound; and

(b) a fluid within the network.

In a fourth aspect, the present invention relates to a personal care composition comprising a branched organosiloxane compound.
In a fifth aspect, the present invention relates to a method for making a personal care composition, comprising combining a personal care ingredient with a branched organosiloxane compound.
In sixth aspect, the present invention relates to a method for improving the sensory feel of a personal care composition while minimizing phase separation of the personal care composition, comprising adding a silicone composition, said silicone composition comprising a network, said network comprising two or more molecules of a branched organosiloxane compound, and a emollient fluid within the network, to the personal care composition.
In a seventh aspect, the present invention is directed to a method for reversibly imparting characteristics of a solid to a fluid, comprising combining the fluid with a branched organosiloxane compound, said branched organosiloxane compound comprising, per molecule of the compound, a silicone core and one or more hydrocarbon terminated branches attached to the silicone core, to form a network comprising two or more molecules of the branched organosiloxane compound with the fluid contained within the network
In its various embodiments, the branched organosiloxane compound of the present invention exhibits a high affinity for a wide variety of fluids, including emollient fluids. The silicone composition of the present invention exhibits good stability, that is, a high resistance to separation of the fluid from the silicone composition. Personal care compositions containing branched organosiloxane compound and an emollient fluid, whether the branched organosiloxane compound and fluid are added separately to the personal care composition or added to the personal care composition in the form of the silicone composition of the present invention, exhibit improved sensory feel, leave a smooth silky feeling in the skin upon dry down, exhibit good film forming ability and exhibit good stability, that is, a high resistance to separation of the emollient fluid from the personal composition.

DETAILED DESCRIPTION OF THE INVENTION

As used herein, the terminology "monoethylenically unsaturated" with respect to a compound means that the compound has one site of ethylenic unsaturation per molecule of the compound and the terminology "polyethylenically unsaturated" with respect to a compound means that the compound contains two or more ethylenically unsaturated sites per molecule of the compound.

In a preferred embodiment, the silicone core of the branched organosiloxane compound of the present invention comprises a silicone resin core. As used herein, the terminology "silicone resin core" means a silicone core comprising one or more siloxane units of the structural formula (I):

$$SiO_{4/2} \qquad (I).$$

In a preferred embodiment, the hydrocarbon terminated branches of the branched organosiloxane compound of the present invention comprise monovalent hydrocarbon radicals that are each covalently bonded, either directly or indirectly, such as, for example, via a divalent organosiloxane group, to a silicon atom of the silicone core of the branched organosiloxane compound of the present invention.

In a highly preferred embodiment, the silicone core of the branched organosiloxane compound of the present invention comprises (a) two or more siloxane nodes, each node comprising one or more siloxane units of the structural formula (I) and (b) one or more organosiloxane bridging groups connecting the siloxane nodes, each organosiloxane bridge comprising one or more organosiloxane units selected from units of one or more of the structural formulas (II), (III) and (IV):

$$R^1_3SiO_{1/2} \qquad (II)$$

$$R^2_2SiO_{2/2} \qquad (III)$$

$$R^3SiO_{3/2} \qquad (IV)$$

wherein each $R^1$, $R^2$ and $R^3$ is independently hydrocarbon radical. In a preferred embodiment, one or more of the $R^1$, $R^2$ and $R^3$ groups represent at least a portion of the hydrocarbon terminated branches of the branched organosiloxane compound of the present invention.

In a preferred embodiment, the branched organosiloxane compound comprises one or more structural units according to formula (II) which are each covalently bonded, either directly or indirectly, such as for example, through a divalent organosiloxane group, to a $SiO_{4/2}$ structural unit of the silicone resin core. In a preferred embodiment, the $R^1$ substituents of such one or more structural units according to formula (II) represent at least a portion of the hydrocarbon terminated branches of the branched organosiloxane compound of the present invention.

As used herein "hydrocarbon radical" includes alicyclic hydrocarbon radicals.

As used herein the term "alkyl" means a saturated straight or branched hydrocarbon radical. In a preferred embodiment, monovalent allyl groups are selected from linear or branched alkyl groups containing from 1 to 80 carbons per group, such as, for example, methyl, ethyl, propyl, iso-propyl, n-butyl, iso-butyl, sec-butyl, tert-butyl, pentyl, hexyl, heptyl, decyl, dodecyl, eicosyl.

As used herein the term "alkenyl" means a straight or branched terminally unsaturated hydrocarbon radical, preferably containing from 2 to 10 carbon atoms per radical, such as, for example, ethenyl, 2-propenyl, 3-butenyl, 5-hexenyl, 7-octenyl and ethenylphenyl.

As used herein, the terminology "alicyclic hydrocarbon radical" means a radical containing one or more saturated hydrocarbon rings, preferably containing from 4 to 10 carbon atoms per ring, per radical which may optionally be substituted on one or more of the rings with one or more alkyl radicals, each preferably containing from 2 to 6 carbon atoms per group, halo radicals or other functional groups and which, in the case of an alicyclic hydrocarbon radical containing two or more rings, may be fused rings. Suitable monovalent alicyclic hydrocarbon radicals include, for example, cyclohexyl and cyclooctyl.

In a preferred embodiment, a hydridosiloxane, preferably, a terminal dihydridosiloxane, is reacted with an ethylenically unsaturated hydrocarbon, preferably a terminally monoethylenically unsaturated hydrocarbon, to produce a reaction intermediate comprising hydrocarbon-substituted siloxane chains. The reaction intermediate is then reacted with a ethylenically unsaturated silicone resin to produce a soluble polymeric system with pendant branches consisting of siloxane chains terminated with the hydrocarbon substituents.

In preferred embodiment, the branched organopolysiloxane compound of the present invention is made by hydrosilylation of an ethylenically unsaturated hydrocarbon and an ethylenically unsaturated siloxane resin with a silylhydride functional organosiloxane, preferably comprising a silylhydride terminated organosiloxane according to the structural formula (VI):

$$M^H{}_a D_b D^H{}_c T_d T^H{}_e \qquad (VI)$$

wherein

M is $R^5{}_3 SiO_{1/2}$

$M^H$ is $HR^6{}_2 SiO_{1/2}$,

D is $R^7{}_2 SiO_{2/2}$,

$D^H$ is $HR^8 SiO_{2/2}$

T is $R^9 SiO_{3/2}$,

$T^H$ is $HSiO_{3/2}$,

wherein each $R^5$, $R^6$, $R^7$, $R^8$ and $R^9$ is independently a hydrocarbon radical and a, b, c, d and e are each integers selected to provide a compound a having a viscosity of from 1 to 1,000,000 cSt, more preferably from 1 to 100,000 cSt, and having a desired amount of silylhydride groups per molecule

In a preferred embodiment, the ethylenically unsaturated hydrocarbon comprises a terminally monoethylenically unsaturated hydrocarbon according to the structural formula (VII):

$$CH_2 = CHR^{10} \qquad (VII)$$

wherein each $R^{10}$ is independently a monovalent hydrocarbon radical.

In a preferred embodiment, the ethylenically unsaturated siloxane resin comprises a polyethylenically unsaturated siloxane resin of the structural formula (VIII):

$$(M^{vi}{}_2 Q)_4 \qquad (VIII)$$

wherein $M^{vi}$ is $R^{11} R^{12}{}_2 SiO_{1/2}$, wherein each $R^{11}$ is independently a monovalent hydrocarbon radical, each $R^{12}$ is alkenyl and Q is $SiO_{4/2}$.

In a preferred embodiment, each $R^5$, $R^6$, $R^7$, $R^8$, $R^9$ and $R^{11}$ is independently alkyl, hydroxyalkyl, a polyhydric alcohol radical, monocyclic aromatic, aralkyl, oxaalkylene or alkylcarbonyloxaalkylene.

More preferably, each $R^5$, $R^6$, $R^7$, $R^8$, $R^9$ and $R^{11}$ is independently $(C_1\text{-}C_{80})$alkyl, hydroxy$(C_1\text{-}C_{12})$alkyl, a polyhydric alcohol radical according to formula (IX), (X) or (XI)

$$- R^{13}\text{-}CHOHCH_2OH \qquad (IX)$$

$$- R^{14}\text{-}CHOHCH_2CH_2OH \qquad (X)$$

$$- R^{15}\text{-}C(R^{16})_3 \qquad (XI)$$

wherein each $R^{13}$, $R^{14}$ and $R^{15}$ is independently $(C_1\text{-}C_{12})$alkylene or $(C_1\text{-}C_{12})$oxaalkylene and each $R^{16}$ is independently H, hydroxy, $(C_1\text{-}C_{12})$alkyl, or hydroxy$(C_1\text{-}C_{12})$alkyl, provided that at least two $R^{16}$ substituents per radical are hydroxy or hydroxy$(C_1\text{-}C_{12})$alkyl,

aralkyl according to the formula (XII):

wherein $R^{17}$ is $(C_1\text{-}C_6)$alkylene and each $R^{18}$ is independently H, hydroxyl, $(C_1\text{-}C_6)$alkyl, hydroxy$(C_2\text{-}C_6)$alkyl, or -$OCOR^{19}$, wherein $R^{19}$ is $(C_1\text{-}C_6)$alkyl,

oxaalkylene according to formula (XIII) or (XIV):

$$-(CH_2)_f O(CR^{20}H)_g - \qquad (XIII)$$

$$-(CH_2)_h (O(CR^{21}H)_i)_j (CH_2)_k - \qquad (XIV)$$

wherein each $R^{20}$ and $R^{21}$ is independently H or alkyl, preferably $(C_1\text{-}C_8)$akyl, and each f, g, h, i, j and k is independently an integer of from 1 to 20, or

alkylcarbonyloxaalkylene according to formula (XV):

$$-R^{22}-C-R^{23}{}_3 \qquad (XV)$$

wherein $R^{22}$ is $(C_1-C_{12})$alkylene or $(C_1-C_{12})$oxaalkylene and each $R^{23}$ is independently H, $(C_1-C_{24})$alkyl, or -$OCOR^{24}$, wherein each $R^{24}$ is independently $(C_1-C_{24})$alkyl, provided that at least one $R^{23}$ group per radical is -$COR^{24}$.

Suitable silylhydride terminated organosiloxanes include, for example, silylhydride terminated polydimethylsiloxanes.

Suitable monoethylenically unsaturated hydrocarbon compounds include, for example, polyolefins, allyl polyethers, allyl esters, vinyl aromatics, monoethylenically unsaturated alcohols.

Suitable polyethylenically unsaturated siloxane resins include, for example, vinyl functional MQ resins.

In one preferred embodiment, the silylhidride terminated organosiloxane compound according to formula (XVI) below is contacted under hydrosilylation conditions with less than its molar equivalent amount, based on relative moles of silylhidride groups and ethylenically unsaturated groups, of a monoethylenically unsaturated hydrocarbon compound according to formula (VII) above to form a reaction intermediate comprising a mixture of products according to the structural formulae (XVI), (XVII) and (XVIII):

$$M^H D_n M^H \qquad (XVI)$$

$$M^1 D_n M^H \qquad (XVII)$$

and

$$M^1 D_n M^1 \qquad (XVIII)$$

wherein $M^1$ is $R^{25}R^6{}_2SiO_{1/2}$, wherein $R^{25}$ is -$(CH_2)_2R^{10}$, and wherein $M^H$, D, $R^6$ and $R^{10}$ are each defined as above, n is an integer selected to provide a compound a having a viscosity of from 1 to 1,000,000 cSt, more preferably from 1 to 100,000 cSt, and the reaction intermediate is then reacted with the ethylenically unsaturated siloxane resin to form a branched organopolysiloxane compound.

In an alternative preferred embodiment, a silylhydride functional organosiloxane is contacted under hydrosilylation conditions with less than its molar equivalent amount of a monoethylenically unsaturated hydrocarbon, wherein the molar equivalent amount is based on relative moles of silylhidride groups of the organosiloxane and ethylenically unsaturated groups of the hydrocarbon, and less than its molar equivalent amount of a polyethylenically unsaturated siloxane resin, wherein the molar equivalent amount is based on relative moles of silylhidride groups of the organosiloxane and ethylenically unsaturated groups of the resin, in a single step to form the branched siloxane compound of the present invention,

In another alternative preferred embodiment, a silylhydride functional organosiloxane contacted under hydrosilylation conditions with less than its molar equivalent amount, based on relative moles of silylhidride groups and ethylenically unsaturated groups, of a polyethylenically unsaturated siloxane resin in a first step and then contacted under hydrosilylation conditions with a monoethylenically unsaturated hydrocarbon to form the branched siloxane compound of the present invention.

The method of polymer synthesis provides for incorporation of a wide range of organofunctional groups into the copolymeric structure. Thus, the inclusion of other organofunctional groups, such as, for example, organic epoxides, epoxysiloxanes, terminally unsaturated organic and alkenylsiloxane compounds can be used to modify the resulting copolymers.

In one embodiment, the organofunctional groups are introduced to the network as $R^5$, $R^6$, $R^7$, $R^8$, $R^9$ and $R^{11}$ radicals present on a silylhydride functional organosiloxane according to formula (VI) or the ethylenically unsaturated siloxane resin according to formula (VIII) above. In an alternative embodiment, the organofunctional groups are introduced to the network during hydrosilylation of the silylhydride functional organosiloxane and the ethylenically unsaturated reactants by including organofunctional compounds, for example ethylenically unsaturated organofunctional groups, to the reaction mixture which are copolymerizable with the silylhydride functional organosiloxane under the chosen polymerization reaction conditions. For example, the silylhydride functional organosiloxane, ethylenically unsaturated hydrocarbon and ethelynicallyunsaturated siloxane resin may be polymerized in the presence of other reactants, such as for example alkenyl functional silicone compounds, alkenyl functional organic compounds or silylhydride functional compounds which contain the desired organodfunctional groups and which areare reactive with or copolymerizable with the silylhydride functional organosiloxane, ethylenically unsaturated hydrocarbon and ethelynicallyunsaturated siloxane resin under the reaction conditions used and the polymer network may, accordingly, include structural units derived from such other reactants.

In a highly preferred embodiment, the branched organosiloxane compound formed by any of the above alternative processes is then treated with a terminally monoethylenically unsaturated hydrocarbon according to structural formula (VI) under hydrosilylation conditions to cap any remaining silylhydride functional groups.

In contrast to the cross-linked, insoluble silicone gel materials, such as, for example, those disclosed in coassigned U.S. Patent No 5,760,116, the branched organosiloxane compound of the present invention has a finite molecular weight and is soluble in, for example, benzene, i.e. the compounds of the present invention are benzene soluble. In preferred embodiment, the branched organosiloxane compound has a number average or weight average molecular weight of less than about 10,000,000, more preferably a number average or weight average molecular weight from about 1,000 to about 10,000,000, even more preferably from about 10,000 to about 5,000,000.

In a preferred embodiment, at least one step of the synthesis of the branched organopolysiloxane compound of the present invention is carried out in the presence of a fluid to produce a network of branched organosiloxane molecules having the fluid within the network.

In an alternative embodiment, the silicone material of the present invention is made by synthesizing the branched organopolysiloxane compound in the absence of fluid, followed by the subsequent addition of a fluid to produce a network of branched organosiloxane molecules having the fluid within the network.

In another alternative embodiment, the silicone material of the present invention is made by synthesizing the branched organopolysiloxane compound of the present invention in the presence of a first fluid such as for example a volatile hydrocarbon fluid, followed by removal of the first fluid, such as, for example by evaporation of the first fluid, and the subsequent addition of a second fluid. such as for example, a siloxane fluid, to produce a network of branched organosiloxane molecules having the second fluid within the network.

As used herein, the terminology "network" means a three dimensionally extending structure comprising two or more molecules of the branched organosiloxane compound. Preferably, fluid is contained within interstices of the network. As used herein, the term "interstices" is used in reference to the network to denote spaces within the network, that is, spaces between the molecules of the branched organosiloxane compound of the network.

The network structure comprises a plurality of molecules of the branched organosiloxane compound, associated via intermolecular attractions between the molecules of the branched organosiloxane compound. Molecules of the branched organosiloxane compound associate to form a network structure when the branched organosiloxane compound is present in a sufficiently high concentration. While not wishing to be bound by theory, it is believed that in those embodiments of the present invention which include waxy hydrocarbon subustituent-terminated branches, the crystallization of the waxy substituent groups of different molecules of the branched organosiloxane compound is the predominant mode of intermolecular attraction that leads to formation of the network. As the concentration of branched organosiloxane compound by diluting the mixture with a suitable fluid, for example, an emollient fluid or a silicone fluid, the magnitude of the intermolecular attractions between the molecules of the branched organosiloxane compound decrease and, at sufficiently high dilution, the mixture forms a solution of the branched organosiloxane compound in the fluid.

In a preferred embodiment, the silicone composition of the present invention comprises, based on 100 parts by weight ("pbw") of the silicone composition, from 0.1 pbw to 99 pbw, more preferably from 1.0 pbw to 90 pbw, even more preferably from 2 pbw to 40 pbw, of the branched organosiloxane compound of the present invention and from 1 pbw to 99.9 pbw, more preferably from 10 pbw to 99 pbw, even more preferably from 60 pbw to 98 pbw, of the fluid.

The silicone composition may be further processed under low to high shear to adjust the viscosity and sensory feel of the composition. This may be achieved, for example, by subjecting the composition to a moderate to high shearing force. High shear may be applied using, for example, a Sonolator apparatus, a Gaulin Homogenizer or a Micro Fluidizer apparatus. Optionally, more fluid may be added prior to the shearing.

In a preferred embodiment, the silicone composition of the present invention is a solid, typically having a creamy consistency, wherein the network acts as a means for reversibly imparting characteristics of a solid to the fluid. At rest, the silicone composition exhibits the properties of a solid. The silicone composition of the present invention exhibits high stability and resistance to syneresis, that is, the composition exhibits little or no tendency for fluid to flow from the composition and imparts high stability and syneresis resistance to personal care compositions which include the silicone composition as a component. The high stability and syneresis resistance persists with prolonged aging of such silicone compositions and personal care compositions. However, fluid may be released from the network by subjecting the silicone composition to a shearing force, such as, for example, by rubbing the composition between one's fingers, to provide improved sensory feel characteristic of the fluid component of the silicone material.

Fluids suitable for use as the fluid component of the composition of the present invention are those compounds or mixtures of two or more compounds that are in the liquid state at or near room temperature, for example, from about 20°C about 50°C, and about one atmosphere pressure, and include, for example, silicone fluids, hydrocarbon fluids, esters, alcohols, fatty alcohols, glycols and organic oils. In a preferred embodiment, the fluid component of the composition of the present invention exhibits a viscosity of below about 1,000 centistokes, preferably below about 500 centistokes, more preferably below about 250 centistokes, and most preferably below 100 centistokes, at 25 °C.

The characterization of one embodiment of the branched organosiloxane compound as being swellable by the fluid means that the embodiment of the branched organosiloxane compound is capable of absorbing the fluid. In a highly preferred embodiment, the composition of the branched organosiloxane compound is tailored to enhance its compatibility with the fluid. For example, if the branched organosilioxane compound network is to be swollen with a hydrocarbon fluid, then the hydrocarbon character of the branched organosiloxane compound may be increased by increasing the number and/ or the carbon chain length of the organic substituents of the polyfunctional organosilicone compound used to form the polymer network.

In a preferred embodiment, the fluid component of the present invention comprises an emollient compound. Suitable emollient compound include any fluid that provides emollient properties, that is, that when applied to skin, tend to remain on the surface of the skin or in the stratum corneum layer of the skin to act as lubricants, reduce flaking and to improve the appearance of the skin. Emollient compounds are generically known and include, for example, hydrocarbons, such as for example, isododecane, isohexadecane, hydrogenated polyisobutene, organic waxes, such as for example, jojoba, silicone fluids, such as, for example, cyclopentasiloxane, dimethicone, bis-phenylpropyl dimethicone, esters, such as, for example, octyldodecyl neopentanoate, oleyl oleate, as well as fatty acids and alcohols, such as for example, oleyl alcohol, isomyristyl alcohol.

In a highly preferred embodiment, the fluid component of the present invention comprises a silicone fluid, more preferably a silicone fluid that exhibits emollient properties. Suitable silicone fluids include, for example, cyclic silicones of the formula Do, wherein D is defined as above, $R^7$ is preferably methyl, and r is an integer wherein $3 \leq 0 \leq 12$, such as, for example, hexamethylcyclotrisiloxane ("$D_3$"), octamethylcyclotetrasiloxane ("$D_4$"), decamethylcyclopentasiloxane ("$D_5$"), and dodecamethylcyclohexasiloxane ("$D_6$"), as well as linear or branched organopolysiloxane fluids according to the formula (XIX):

$$M'_p D'_q T'_r \, x \qquad (XVIII)$$

wherein:

M' is $R^{26}_3 SiO_{1/2;}$

D' is $R^{27}_2 SiO_{2/2}$

T' is $R^{28} SiO_{3/2}$

$R^{26}$, $R^{27}$ and $R^{28}$ are each independently alkyl, aryl or aralkyl;

p, q and r are zero or positive integers, wherein p = (2 +r) , $0 \leq q \leq 300$, and when p and r are zero, q is 3 or greater; preferably $0 \leq q \leq 100$,

more preferably $0 \leq q \leq 50$, and even more preferably $0 \leq q \leq 20$, and $0 \leq r \leq 100$.

The personal care applications where the branched organosiloxane compound of the present invention and the silicone composition of the present invention may be employed include, but are not limited to, deodorants, antiperspirants, antiperspirant/deodorants, shaving products, skin lotions, moisturizers, toners, bath products, cleansing products, hair care products such as shampoos, conditioners, mousses, styling gels, hair sprays, hair dyes, hair color products, hair bleaches, waving products, hair straighteners, manicure products such as nail polish, nail polish remover, nails creams and lotions, cuticle softeners, protective creams such as sunscreens, insect repellents and anti-aging products, color cosmetics such as lipsticks, foundations, face powders, eye liners, eye shadows, blushes, makeup, mascaras and other personal care formulations where silicone components have been conventionally been added, as well as drug delivery systems for topical application of medicinal compositions that are to be applied to the skin.

In a preferred embodiment, the personal care composition of the present invention comprises one or more personal care ingredients. Suitable personal care ingredients include, for example, emollients, including, for example, the emollient fluids discussed above, moisturizers, humectants, water soluble dyes, liposoluble dyes, pigments, including pearlescent pigments such as, for example, bismuth oxychloride and titanium dioxide coated mica, colorants, fragrances, biocides, preservatives, antioxidants, antimicrobial agents, anti-fungal agents, antiperspirant agents, exfoliants, hormones, enzymes, medicinal compounds, vitamins, salts, electrolytes, alcohols, polyols, absorbing agents for ultraviolet radiation, botanical extracts, surfactants, silicone oils, organic oils, waxes, film formers, thickening agents such as, for example, fumed silica or hydrated silica, particulate fillers, such as for example, silica, talc, kaolin, starch, modified starch, mica, nylon, polyethylene powder, poly(methyl methacrylate) powder and clays, such as, for example, bentonite and organo-modified clays.

Suitable personal care compositions are made by combining, in a manner known in the art, such as, for example, by mixing, one or more of the above components with the silicone network of the present invention or with the silicone composition of the present invention. Suitable personal care compositions may be in the form of a single phase or in the form of an emulsion, including oil-in-water, water-in-oil and anhydrous emulsions, as well as multiple emulsions, such as, for example, oil-in water-in-oil emulsions and water-in-oil-in water-emulsions.

In a preferred embodiment, an antiperspirant composition comprises a silicone material according to the present invention and one or more active antiperspirant agents. Suitable antiperspirant agents include, for example, the Category I active antiperspirant ingredients listed in the U.S. Food and Drug Administration's October 10, 1993 Monograph on antiperspirant drug products for over-the-counter human use, such as, for example, aluminum halides, aluminum hydroxyhalides, for example, aluminum chlorohydrate, and complexes or mixtures thereof with zirconyl oxyhalides and zirconyl hydroxyhalides, such as for example, aluminum-zirconium chlorohydrate, aluminum zirconium glycine complexes, such as, for example, aluminum zirconium tetrachlorohydrexgly.

In a preferred embodiment, a skin care composition comprises silicone material of the present invention and a vehicle, such as, for example, a silicone oil or an organic oil. The skin care composition may, optionally, further include emollients, such as, for example, triglyceride esters, wax esters, alkyl or alkenyl esters of fatty acids or polyhydric alcohol esters and one or more the known components conventionally used in skin care compositions, such as, for example, pigments, vitamins, such as, for example, Vitamin A, Vitamin C and Vitamin E, sunscreen or sunblock compounds, such as, for example, titanium dioxide, zinc oxide, oxybenzone, octylmethoxy cinnamate, butylmethoxy dibenzoylmethane, p-aminobenzoic acid and octyl dimethyl-p-aminobenzoic acid.

In a preferred embodiment, a color cosmetic composition, such as, for example, a lipstick, a makeup or a mascara composition comprises a silicone material according to the present invention, an emollient compound and one or more coloring agents, such as, for example, pigments, water soluble dyes or liposoluble dyes.

The compositions of the present invention may be utilized directly as silicone compositions or as emulsions. As emulsions they may be utilized as silicone in water (oil-in-water) emulsions or as water in silicone (water-in-oil) emulsions. They may also be utilized as non-aqueous emulsions between immiscible non-aqueous phases where the silicone comprising phase is the discontinuous phase of the emulsion or where the silicone comprising phase is the continuous phase of the emulsion. Non-aqueous emulsions comprising a silicone phase are described in US patent 6,060,546 and co-pending application US Ser. No. 09/033,788 filed March 3, 1998. As used herein the term "emulsion" includes but is not limited to microemulsions and emulsions within emulsions.

The following examples are by way of illustration only and are not intended to limit the appended claims in any fashion.

Example 1 (reference example)

A branched organopolysiloxane compound of the present invention was made as follows. 7.5 grams (0.00797 moles) of terminally unsaturated ($C_{30+}$)hydrocarbon wax (Gulftene 30+, Chevron) was mixed with 100 grams (0.0207 moles) of a silylhydride terminated organosiloxane of the structural formula $M^H D_{125} M^H$, wherein $M^H$ and D are each defined as above and $R^4$ and $R^5$ are each methyl, and 0.00504 grams of 10% platinum catalyst. The mixture was heated for two hours at 80°C to allow the wax to react on the ends of the silylhydride fluid. An amount (0.97 grams) of an organosiloxane resin according to the structural formula $(M^{vi}_2 Q)_4$, wherein $M^{vi}$ and Q are each defined above, $R^8$ is ethenyl and $R^9$ is methyl, to provide 0.0093 moles of vinyl equivalents was then added to the reaction mixture along with an additional 0.00504 grams of platinum catalyst. The reaction mixture was again heated to 90-95°C for two hours to allow reaction. An additional 3.23 grams of the terminally unsaturated wax (0.0062 moles) was then added and again the reaction was heated to 90-95°C for two hours. The reaction product was a high viscosity, flowable liquid that solidified on cooling. An SiH test showed that the SiH had been consumed during the reaction.

Example 2 (reference example) and Comparative Example C1

The antiperspirant compositions of Example 2 and Comparative Example C1 were made by combining the ingredients set forth below in the relative amounts listed in TABLE I below. Unless otherwise specified, all the relative amounts of the ingredients of the compositions of the examples and comparative examples disclosed below are given in pbw per 100 pbw of the composition, with the notation "q.s." used with some ingredients, for example, a fragrance, where the amount of the ingredients is not critical, to indicate a non-measured sufficient amount of the ingredient

TABLE I

| Ingredients | CEx.C1 | Ex 2 |
|---|---|---|
| ($C_{30}$-$C_{45}$)allyl dimethicone wax | 6 | -- |
| Compound of Example 1 | -- | 6 |

(continued)

| Ingredients | CEx.C1 | Ex 2 |
|---|---|---|
| Cyclopentasiloxane | 45 | 45 |
| Hydrogenated castor oil | 5 | 5 |
| $(C_{12}$-$C_{15})$alkyl benzoate | 14 | 14 |
| Talc | 5 | 5 |
| ZAG | 25 | 25 |

The antiperspirant compositions of Example 2 and Comparative Example C1 were each evaluated for evidence of syneresis by maintaining the compositions at room temperature, with syneresis indicated by phase separation. Neither composition showed any evidence of syneresis after one week.

The spreadability of the each of compositions of Example 2 and Comparative Example C1 were evaluated by spreading the samples on the forearms of a test subject and evaluating the ease of moving the composition over the skin surface. Both compositions exhibited high spreadability.

The composition of Example 2 provided softer feel during rub on when compared to the composition of Comparative Example C1.

Example 3 (reference example) and Comparative Example C2

The white antiperspirant sticks of Example 3 and Comparative Example C2 were made by combining the ingredients in the relative amounts set forth below in Table II. The cyclopentasiloxane, stearyl alcohol, hydrogenated castor oil, PPG-2 myristyl ether proprionate and in Example 3, the material of Example 1, were combined and heated to 65°C. Talc and ZAG were then added to the heated mixture which was then mixed until uniform and poured into containers.

TABLE II

| Ingredients | CEx C2 | EX 3 |
|---|---|---|
| Cyclopentasiloxane | 54 | 49 |
| Compound of Example 1 | -- | 5 |
| ZAG | 24 | 24 |
| Stearyl alcohol | 14 | 14 |
| PPG-2 myristyl ether proprionate | 1 | 1 |
| Talc | 3 | 3 |
| Hydrogenated castor oil | 4 | 4 |

After 24 hours, the antiperspirant sticks were evaluated for an anti-whitening effect and the ability to hold cyclopentasiloxane and PPG-2 myristyl ether proprionate into the stick. The test for the anti-whitening effect was performed by applying 200 mg of antiperspirant (formulations listed above) on 5cm x11cm black tiles. A Minolta CR300 Colorimeter was used to quantify the whitening after 2 hours by measuring L- values on L,a,b color scale which represents whiteness.

$$\% \text{ whitening reduction} = (L_0 - L) / L_0 \times 100$$

where $L_0$ is L value of control(C2), and L is L value of the tested formulation (Example 3). The antiperspirant stick of Example 3 showed % whitening reduction of 43 % ($L_0 = 58.69$, L= 33.57).

The ability of each of the antiperspirant sticks to hold cyclopentasiloxane and PPG-2 myristyl ether proprionate in the respective stick was performed by pressing a thumb on the surface of the stick and observed the fluid squeezing out. The antiperspirant stick of Example 3 showed no leakage whereas the antiperspirant stick of Comparative Example C2 showed the fluid weeping. The antiperspirant stick of Example 3 was also harder than that of C2. This indicates that material Example 1 has the ability to hold emollient fluids in addition to its gelling property. Both samples were applied on skin. The antiperspirant stick of Example 3 provided superior glide compared to that of Comparative Example C2. In general, superior glide results in a uniform active salt deposition on skin.

Example 4 (reference example) and Comparative Example C3

The oil-in-water emulsion compositions of Example 4 and Comparative Example C3, each useful, for example, as a skin lotion, were made by combining the ingredients in the relative amounts listed below in Table III and assessing skin feel.

TABLE III

| Ingredients | CEx C3 | Ex 4 |
|---|---|---|
| Part A | | |
| Water | 76.4 | 71.4 |
| Disodium EDTA | 0.05 | 0.05 |
| Methylparaben | 0.2 | 0.2 |
| Propylparaben | 0.1 | 0.1 |
| 2% Carbomer (Carbopol 934) | 20 | 20 |
| | | |
| Part B | | |
| Glyceryl stearate and PEG-100 stearate (Arlacel165) | 1.6 | 1.6 |
| Vitamin E | 0.5 | 0.5 |
| Compound of Example 1 | -- | 5 |
| | | |
| Part C | | |
| DI Water | 1 | 1 |
| 99% TEA | 0.15 | 0.15 |

Each of the emulsion compositions of Example 4 and Comparative Example C3 was made by: (1) heating Part A and Part B in separate vessels to 70°C with moderate agitation, (2) adding Part B to Part A under homogenization, (3) cooling the mixture so formed to 40°C and adding Part C as ordered, and (4) pouring the cooled mixture into containers.
Sensory evaluation was performed on both samples by rubbing samples on skin. Initial feel was similar, but the emulsion of Example 4 provided better spreadability and a more silky feel upon rubbing than that of Comparative Example C3.

Example 5 (reference example) and Comparative Example C4

Lipstick examples comprising the ingredients listing below were made by combining the ingredients in the relative amounts set forth below in Table IV. The cyclopentasiloxane, cetearyl methicone or silicone composition of Example 1 and the pigment were combined and heated to 65°C. The mica was then added to the heated mixture and mixed well.

TABLE IV

| Ingredients | CEx C4 | Ex 5 |
|---|---|---|
| Cyclopenatasiloxane | 50 | 50 |
| Compound of Example 1 | 0 | 40 |
| Cetearyl methicone | 40 | 0 |
| D&C Red #7 Ca lake | 8 | 8 |
| Mica | 2 | 2 |

Each of the compositions were evaluated for appearance and durability after 24 hour. The lipstick of Comparative Example 4 formed a stick, whereas The lipstick of Example 5 was a soft solid lipstick with a deeper color and more glossy. Both samples were then tested for durability by applying material on 5cm x11cm black tiles. Then the tiles were rinsed with water for 60 sec and the amount of material left on each tile was evaluated. The lipstick of Comparative Example C4 was washed away clean, while the lipstick of Example 5 remained on the tile, showing high water repellency and durability.

Example 6 (reference example) and Comparative example C5

The water-in-oil emulsion compositions of Example 6 and Comparative Example C5 were made by combining the ingredient in the relative amounts set forth below in Table V. Parts A and B were separately prepared and then combined.

TABLE V

| Ingredients | CEx C5 | Ex 6 |
| --- | --- | --- |
| Part A | | |
| Cyclopentasiloxane and Dimethicone Copolyol | 10 | 10 |
| Cyclopentasiloxane | 16 | 8 |
| Compound of Example 1 | -- | 8 |
| Sorbitan oleate | 0.6 | 0.6 |
| Part B | | |
| Glycerin | 1 | 1 |
| NaCl | 1 | 1 |
| Germaben II | 1 | 1 |
| Water | 70.4 | 70.4 |

The appearance, thickening effect and skin feel of the emulsions were then evaluated. Sensory evaluation was conducted by applying skin cream on forearm and assessing skin feel of the composition of Example 6 compared to that of Comparative Example C5. The thickening effect was identified by measuring viscosity of the formulations after 24 hour by using a Brookfield viscometer with a t-spindle and heliopath stand. Results of the evaluations are set forth below in Table VI.

TABLE VI

| | CEx C5 | Ex 6 |
| --- | --- | --- |
| Appearance after 24 hr at RT | Pourable lotion | Thick cream |
| Viscosity at 25°C, centiPoise | 11,232 | 374,400 |
| Skin feel | Initial light feel during rub in, and high spreadability | Substantive soft smooth feel after water evaporated off, and moderate spreadability |

Example 7 (reference example)

The sunscreen lotion composition of Example 7 is made by combining the ingredients in the relative amounts set forth below in Table VII according to the procedure outlined below and gives good skin feel and water repellency.

TABLE VII

| Ingredient | Relative Amount |
| --- | --- |
| PART A | |
| Deionized Water | q.s. |
| Tetrasodium EDTA | 0.05 |
| PEG-8 | 4.00 |
| Phenoxyethanol, Methylparaben, Butylparaben, Ethylparaben and Propylparaben | 0.25 |
| Magnesium Aluminum Silicate | 0.25 |

(continued)

| Ingredient | Relative Amount |
|---|---|
| PART B | |
| Compound of Example 1 | 7.00 |
| Octyl Methoxycinnamate | 7.00 |
| Octyl Salicylate | 3.00 |
| Benzophenone-3 | 3.00 |
| $(C_{10-30})$alkylacrylate Crosspolymer | 0.30 |
| Carbomer (Carbopol 934) | 0.15 |
| Sorbitan Oleate | 0.20 |
| | |
| PART C | |
| Fragrance | 0.12 |
| | |
| PART D Triethanolamine 99% | |
| | 0.55 |

The ingredients are combined according to the following procedure: (1) make Part A by (a) heating water of Part A to 75°C, (b) adding remaining ingredients in order with moderate propeller agitation, making sure that all paraben components have dissolved, (c) mixing for 15 minutes, while cooling to 50°C, (2) combine the ingredients of Part B with sweep agitation at ambient temperature and mix until a smooth "paste" is obtained, (3) add Part B at room temperature to Part A (at 50°C) with rapid propeller agitation and mix for 30. minutes or longer to ensure that the polymers are completely dispersed, (4) cool with agitation to 45°C. (5) add Part C to batch with moderate propeller agitation and mix 10 minutes, and (6) add Part D to batch at 40°C, mix with moderate agitation for 20 minutes and cool to room temperature.

Example 8 (reference example)

The foundation composition of Example 8 is made by combining the ingredients in the relative amounts set forth below in Table IX according to the procedure set forth below provides superior for long wear and silky light feel.

TABLE IX

| Ingredient | Relative Amount |
|---|---|
| PART A | |
| Cyclopentasiloxane and Dimethicone Copolyol | |
| | 12.0 |
| Cyclopentasiloxane | 20.0 |
| Compound of Example 1 | 5.0 |
| Polymethylsilsesquioxane (TOSPEARL® 2000) | 2.0 |
| Titanium Dioxide | 6.0 |
| Iron Oxides | 2.1 |
| | |
| PART B | |
| 1% NaCl in Deionized Water | 49.95 |
| Polysorbate-20 | 0.85 |
| Glycerin | 2.0 |
| Preservative | q.s. |
| Fragrance | q.s. |

The ingredients are combined by the following procedure: (1) combine the ingredients of Part A, in order shown, thoroughly mixing each component until homogenous before adding the next ingredient, (2) in a separate vessel, combine ingredients of Part B in order shown, (3) slowly add Part B to Part A with good mixing. Increase agitation, as mixture thickens.

Example 9 (reference example)

The shampoo composition of Example 9 is made by combining the ingredients in the relative amounts set forth below in Table X according to the procedure set forth below provides good conditioning to hair fibers.

TABLE X

| Ingredient | Relative Amount |
| --- | --- |
| PART A | |
| Deionized water | q.s. |
| Ammonium Lauryl Sulfate (26%) | 24.00 |
| Ammonium Laureth Sulfate (28%) | 14.30 |
| Cocamidopropyl Betaine | 6.00 |
| | |
| Part B | |
| Cocamide MEA | 4.00 |
| PEG-150 Pentaerythrityl Tetrastearate | 1.50 |
| Compound of Example 1 | 2.5 |
| | |
| Part C | |
| Methylchloroisothiazolinone and | |
| Methylisothiazolinone | 0.05 |
| Citric acid | Adjust to pH 6.0-6.5 |

The ingredients are combined according to the following procedure: (1) heat together all ingredients of Part A at 65°C with moderate agitation, (2) melt Part B in a separate container and add to Part A when melted, (3) cool mixtures to 40°C and add Part C in the order listedand (4) adjust pH to 6.0-6.5 by addition of sufficient amount of citric acid.

Example 10 (reference example)

The leave-in conditioner composition of Example 10 is made by combining the ingredients in Table XI according to the procedure set forth below and can be sprayed onto the hair and used throughout the day to provide shine and conditioning.

TABLE XI

| Ingredient | Relative Amount |
| --- | --- |
| Phenyl Trimethicone | 10.0 |
| Cyclopentasiloxane | 85.0 |
| Compound of Example 1 | 5.0 |

The ingredients are combined according to the following procedure: (1) mix together phenyl trimethicone and cyclopentasiloxane until uniform, and (2) add the silicone composition of Example 1 with stirring.

Example 11 (reference example)

The rinse-off conditioner composition of Example 11 is made by combining the ingredients set forth below in Table XII according to the procedure set forth below and gives excellent conditioning effects to hair which are soft, smooth, and silky feel.

TABLE XII

| Ingredient | Relative Amount |
| --- | --- |
| Part A | |
| Deionized water | q.s. |

(continued)

| Ingredient | Relative Amount |
|---|---|
| Hydroxyethylcellulose | 0.50 |
| Glycerin | 2.00 |
| Methylparaben | 0.20 |
| Propylparaben | 0.10 |
| Part B | |
| Cetearyl alcohol, | |
| Dicetyldimonium Chloride and | |
| Stearamidopropyl Dimethylamine | 3.00 |
| Glyceryl Stearate | 0.80 |
| Compound of Example 1 | 3.00 |
| Cetyl Alcohol | 1.50 |
| Part C | |
| Methylchloroisothiazolinone(and) | 0.05 |
| Methylisothiazolinone | |

The ingredients are combined according to the following procedure: (1) heat together all ingredients of Part A at 65°C, (2) melt Part B in a separate container and add to Part A when melted, and (3) cool mixtures to 40°C and add Part C in the order listed.

The branched organosiloxane compound of the present invention exhibits a high affinity for a wide variety of fluids, including emollient fluids. The silicone composition of the present invention exhibits good stability, that is, a high resistance to separation of the fluid from the silicone composition. Personal care compositions containing branched organosiloxane compound and an emollient fluid, whether the branched organosiloxane compound and fluid are added separately to the personal care composition or added to the personal care composition in the form of the silicone composition of the present invention, exhibit improved sensory feel, leave a smooth silky feeling in the skin upon dry down, exhibit good film forming ability and exhibit good stability, that is, a high resistance to separation of the emollient fluid from the personal composition.

**Claims**

1. A branched organosiloxane silicone composition comprising a silicone resin core wherein said silicone resin core comprises:

   (a) two or more siloxane units of the structural formula (I):

   $$SiO_{4/2} \qquad (I)$$

   wherein said siloxane units are covalently bonded, either directly to each other or indirectly via one or more bridging organosiloxane groups and
   (b) one ore more terminal groups having the structural formula (II):

   $$R^1_3SiO_{1/2} \qquad (II)$$

   wherein said bridging organosiloxane groups are selected from the group of organosiloxanes having the structural formulas (III) and (IV):

   $$R^2_2SiO_{2/2} \qquad (III)$$

   $$R^3SiO_{3/2} \qquad (IV)$$

   wherein each $R^1$ in each terminal group (II), each $R^2$ in each bridging group (III) and each $R^3$ in each bridging group (IV) is independently a hydrocarbon radical,

**characterized in that**
each $R^1$ in each terminal group (II), each $R^2$ in each bridging group (III) and each $R^3$ in each bridging group (IV) is independently an alicyclic hydrocarbon radical.

2. A branched organosiloxane silicone composition comprising a network wherein said network comprises:

(a) a silicone resin core wherein said silicone resin core comprises:

(i) two or more siloxane units of the structural formula (I):

$$SiO_{4/2} \qquad (I)$$

wherein said siloxane units are covalently bonded, either directly to each other or indirectly via one or more bridging organosiloxane groups and
(ii) one or more terminal groups having the structural formula (II):

$$R^1{}_3SiO_{1/2} \qquad (II)$$

wherein said bridging organosiloxane groups are selected from the group of organosiloxane having the structural formulas (III) and (IV):

$$R^2{}_2SiO_{2/2} \qquad (III)$$

$$R^3SiO_{3/2} \qquad (IV)$$

wherein each $R^1$ in each terminal group (I), each $R^2$ in each bridging group (III) and each $R^3$ in each bridging group (IV) is independently a hydrocarbon radical and
(b) a fluid within the network,
**characterized in that**
each $R^1$ in each terminal group (II), each $R^2$ in each bridging group (III) and each $R^3$ in each bridging group (IV) is independently an alicyclic hydrocarbon radical.

3. The composition of claim 2 wherein said fluid within said network is a linear branched or cyclic organopolysiloxane fluid according to the formula (XIX):

$$M'_pD'_qT'_r \qquad (XIX)$$

wherein:

M' is $R^{26}{}_3SiO_{1/2}$;
D' is $R^{27}{}_2SiO_{2/2}$
T' is $R^{28}SiO_{3/2}$
$R^{26}$, $R^{27}$ and $R^{28}$ are each independently alkyl, aryl or aralkyl;

p, q and r are zero or positive integers, $0 \le q \le 300$, and when p and r are both zero, q is 3 or greater.

4. The composition of any preceding claim where at least one of $R^1$, $R^2$ and $R^3$ is selected from the group of hydrocarbon radicals having from 1 to 70 carbon atoms per radical and where at least one of $R^1$, $R^2$ and $R^3$ is selected from the group of hydrocarbon radicals having from 10 to 80 carbon atoms per radical.

5. The composition of claim 4 where at least one of $R^1$, $R^2$ and $R^3$ is selected from the group of hydrocarbon radicals having from 1 to 40 carbon atoms per radical and where at least one of $R^1$, $R^2$ and $R^3$ is selected from the group of hydrocarbon radicals having from 40 to 80 carbon atoms per radical.

6. The composition of any preceding claim wherein said silicone is benzene soluble.

7. The composition of any preceding claim wherein said silicone has a viscosity ranging from 1 to 1,000,000 cSt.

8. A water-in-oil emulsion, an oil-in-water emulsion or a non-aqueous emulsion comprising the composition of any preceding claim.

9. A cosmetic composition comprising a branched organosiloxane silicone composition comprising a silicone resin core wherein said silicone resin core comprises:

(a) two or more siloxane units of the structural formula (I):

$$SiO_{4/2} \qquad (I)$$

wherein said siloxane units are covalently bonded, either directly to each other or indirectly via one or more bridging organosiloxane groups and
(b) one ore more terminal groups having the structural formula (II):

$$R^1{}_3SiO_{1/2} \qquad (II)$$

wherein said bridging organosiloxane groups are selected from the group of organosiloxanes having the structural formulas (III) and (IV):

$$R^2{}_2SiO_{2/2} \qquad (III)$$

$$R^3SiO_{3/2} \qquad (IV)$$

wherein each $R^1$ in each terminal group (II), each $R^2$ in each bridging group (III) and each $R^3$ in each bridging group (IV) is independently a hydrocarbon radical,
**characterized in that**
each $R^1$ in each terminal group (II), each $R^2$ in each bridging group (III) and each $R^3$ in each bridging group (IV) is independently an alicyclic hydrocarbon radical.

**Patentansprüche**

1. Verzweigte Organosiloxansilikonzusammensetzung, welche einen Silikonharzkern umfasst, **dadurch gekennzeichnet, dass** der Silikonharzkern umfasst:

(a) zwei oder mehr Siloxaneinheiten der Strukturformel (I):

$$SiO_{4/2} \qquad (I)$$

wobei die Siloxaneinheiten kovalent verbunden sind, entweder direkt miteinander oder indirekt mittels einer oder mehrere Brückenorganosiloxangrupppen und
(b) eine oder mehr endständige Gruppen, welche die Strukturformel (II) aufweisen:

$$R^1{}_3SiO_{1/2} \qquad (II)$$

wobei die Brückenorganosiloxangruppen aus der Gruppe von Organosiloxanen ausgewählt sind, welche die Strukturformeln (III) und (IV) aufweisen:

$$R^2{}_2SiO_{2/2} \qquad (III)$$

$$R^3SiO_{3/2} \qquad (IV)$$

wobei jedes $R^1$ in jeder endständigen Gruppe (II), jedes $R^2$ in jeder Brückengruppe (III) und jedes $R^3$ in jeder Brückengruppe (IV) unabhängig ein Kohlenwasserstoffradikal ist,

**dadurch gekennzeichnet, dass**
jedes $R^1$ in jeder endständigen Gruppe (II), jedes $R^2$ in jeder Brückengruppe (III) und jedes $R^3$ in jeder Brückengruppe

(IV) unabhängig ein alizyklisches Kohlenwasserstoffradikal ist.

2. Verzweigte Organosiloxansilikonzusammensetzung, welche ein Netzwerk umfasst, **dadurch gekennzeichnet, dass** das Netzwerk umfasst:

(a) einen Silikonharzkern, wobei der Silikonharzkern umfasst:

(i) zwei oder mehr Siloxaneinheiten der Strukturformel (I):

$$SiO_{4/2} \qquad (I)$$

wobei die Siloxaneinheiten kovalent verbunden sind, entweder direkt miteinander oder indirekt mittels einer oder mehrerer Brückenorganosiloxangruppen, und
(ii) eine oder mehrere endständige Gruppen die Strukturformel (II) aufweisen:

$$R^1_3SiO_{1/2} \qquad (II)$$

wobei die Brückenorganosiloxangruppen aus der Gruppe von Organosiloxan ausgewählt sind, welche die Strukturformeln (III) und (IV) aufweist:

$$R^2_2SiO_{2/2} \qquad (III)$$

$$R^3SiO_{3/2} \qquad (IV)$$

wobei jedes $R^1$ in jeder endständigen Gruppe (I), jedes $R^2$ in jeder Brückengruppe (III) und jedes $R^3$ in jeder Brückengruppe (IV) unabhängig ein Kohlenwasserstoffradikal ist und
(b) ein Fluid in dem Netzwerk,

**dadurch gekennzeichnet, dass**
jedes $R^1$ in jeder endständigen Gruppe (II), jedes $R^2$ in jeder Brückengruppe (III) und jedes $R^3$ in jeder Brückengruppe (IV) unabhängig ein alizyklisches Kohlenwasserstoffradikal ist.

3. Zusammensetzung nach Anspruch 2, **dadurch gekennzeichnet, dass** das Fluid in dem Netzwerk ein lineares verzweigtes oder ein zyklisches Organopolysiloxanfluid gemäß der Formel (XIX) ist:

$$M'_pD'_qT'_r \qquad (XIX)$$

wobei:

M' $R^{26}_3SiO_{1/2}$ ist;
D' $R^{27}_2SiO_{2/2}$ ist;
T' $R^{28}SiO_{3/2}$ ist;
$R^{26}$, $R^{27}$ und $R^{28}$ jeweils unabhängig Alkyl, Aryl oder Aralkyl sind;

p, q und r Null oder positive ganze Zahlen, $0 \le q \le 300$ und, wenn p und r beide Null sind, q 3 oder größer ist.

4. Zusammensetzung nach einem der vorstehenden Ansprüche, wobei mindestens eines von $R^1$, $R^2$ und $R^3$ aus der Gruppe von Kohlenwasserstoffradikalen, welche 1 bis 70 Kohlenstoffatome pro Radikal aufweisen, ausgewählt ist und wobei mindestens eines von $R^1$ $R^2$ und $R^3$ aus der Gruppe von Kohlenwasserstoffradikalen, welche 10 bis 80 Kohlenstoffatome pro Radikal aufweisen, ausgewählt ist.

5. Zusammensetzung nach Anspruch 4, wobei mindestens eines von $R^1$, $R^2$ und $R^3$ aus der Gruppe von Kohlenwasserstoffradikalen, welche 1 bis 40 Kohlenstoffatome pro Radikal aufweisen, ausgewählt ist und wobei mindestens eines von $R^1$, $R^2$ und $R^3$ aus der Gruppe von Kohlenwasserstoffradikalen, welche 40 bis 80 Kohlenstoffatome pro Radikal aufweisen, ausgewählt ist.

6. Zusammensetzung nach einem der vorstehenden Ansprüche, **dadurch gekennzeichnet, dass** das Silikon benzenlöslich ist.

7.  Zusammensetzung nach einem der vorstehenden Ansprüche, **dadurch gekennzeichnet, dass** das Silikon eine Viskosität in einem Bereich von 1 bis 1.000.000 cSt. aufweist.

8.  Wasser-in-Öl-Emulsion, Öl-in-Wasser-Emulsion oder nichtwässrige Emulsion, welche die Zusammensetzung nach einem der vorstehenden Ansprüche umfasst.

9.  Kosmetikzusammensetzung, welche eine verzweigte Organosiloxansilikonzusammensetzung umfasst, welche einen Silikonharzkern umfasst, wobei der Silikonharzkern umfasst:

    (a) zwei oder mehr Siloxaneinheiten der Strukturformel (I):

    $$SiO_{4/2} \qquad \text{(I)}$$

    wobei die Siloxaneinheiten kovalent verbunden sind, entweder direkt miteinander oder indirekt mittels einer oder mehrerer Brückenorganosiloxangruppen und
    (b) eine oder mehrere endständige Gruppen, welche die Strukturformel (II) aufweisen:

    $$R^1_3SiO_{1/2} \qquad \text{(II)}$$

    wobei die Brückenorganosiloxangruppen aus der Gruppe aus Organosiloxanen ausgewählt sind, welche die Strukturformeln (III) und (IV) aufweisen:

    $$R^2_2SiO_{2/2} \qquad \text{(III)}$$

    $$R^3SiO_{3/2} \qquad \text{(IV)}$$

    wobei jedes $R^1$ in jeder endständigen Gruppe (II), jedes $R^2$ in jeder Brückengruppe (III) und jedes $R^3$ in jeder Brückengruppe (IV) unabhängig ein Kohlenwasserstoffradikal ist,
    **dadurch gekennzeichnet, dass**
    jedes $R^1$ in jeder endständigen Gruppe (II), jedes $R^2$ in jeder Brückengruppe (III) und jedes $R^3$ in jeder Brückengruppe (IV) unabhängig ein alizyklisches Kohlenwasserstoffradikal ist.

## Revendications

1.  Composition de silicone d'organosiloxane ramifiée comprenant un noyau en résine silicone, où ledit noyau en résine silicone comprend:

    (a) deux ou plusieurs unités de siloxane de la formule structurelle (I):

    $$SiO_{4/2} \qquad \text{(I)}$$

    où lesdites unités de siloxane sont liées de manière covalente, soit directement les unes aux autres soit indirectement par un ou plusieurs groupes d'organosiloxane de pontage et
    (b) un ou plusieurs groupes terminaux présentant la formule structurelle (II):

    $$R^1_3SiO_{1/2} \qquad \text{(II)}$$

    où lesdits groupes d'organosiloxanes de pontage sont sélectionnés dans le groupe d'organosiloxanes ayant les formules structurelles (III) et (IV):

    $$R^2_2SiO_{2/2} \qquad \text{(III)}$$

    $$R^3SiO_{3/2} \qquad \text{(IV)}$$

où chaque $R^1$ dans chaque groupe terminal (II), chaque $R^2$ dans chaque groupe de pontage (III) et chaque $R^3$ dans chaque groupe de pontage (IV) est indépendamment un radical d'hydrocarbure,

**caractérisée en ce que**
chaque $R^1$ dans chaque groupe terminal (II), chaque $R^2$ dans chaque groupe de pontage (III) et chaque $R^3$ dans chaque groupe de pontage (IV) est indépendamment un radical d'hydrocarbure alicyclique.

2. Composition de silicone d'organosiloxane ramifiée comprenant un réseau, où ledit réseau comprend:

   (a) un noyau de résine silicone, où ledit noyau de résine silicone comprend:

   (i) deux ou plusieurs unités de siloxane de la formule structurelle (I):

   $$SiO_{4/2} \qquad (I)$$

   où lesdites unités de siloxane sont liées de manière covalente, soit directement les unes aux autres soit indirectement par un ou plusieurs groupes d'organosiloxane de pontage et
   (ii) un ou plusieurs groupes terminaux ayant la formule structurelle (II):

   $$R^1{}_3SiO_{1/2} \qquad (II)$$

   où lesdits groupes d'organosiloxanes de pontage sont sélectionnés dans le groupe d'organosiloxane ayant les formules structurelles (III) et (IV):

   $$R^2{}_2SiO_{2/2} \qquad (III)$$

   $$R^3SiO_{3/2} \qquad (IV)$$

   où chaque $R^1$ dans chaque groupe terminal (I), chaque $R^2$ dans chaque groupe de pontage (III) et chaque $R^3$ dans chaque groupe de pontage (IV) est indépendamment un radical d'hydrocarbure et
   (b) un fluide dans le réseau,
   **caractérisée en ce que**
   chaque $R^1$ dans chaque groupe terminal (II), chaque $R^2$ dans chaque groupe de pontage (III) et chaque $R^3$ dans chaque groupe de pontage (IV) est indépendamment un radical d'hydrocarbure alicyclique.

3. Composition selon la revendication 2, où ledit fluide dans ledit réseau est un fluide d'organopolysiloxane linéaire, ramifié ou cyclique selon la formule (XIX):

   $$M'_pD'_qT'_r \qquad (XIX)$$

   où:

   M' est $R^{26}{}_3SiO_{1/2}$;
   D' est $R^{27}{}_2SiO_{2/2}$
   T' est $R^{28}SiO_{3/2}$
   $R^{26}$, $R^{27}$ et $R^{28}$ sont chacun indépendamment alkyle, aryle ou aralkyle;

   p, q et r sont zéro ou des entiers positifs, $0 \leq q \leq 300$, et lorsqu'à la fois p et r sont zéro, q est 3 ou plus grand.

4. Composition selon l'une quelconque des revendications précédentes, où au moins un parmi $R^1$, $R^2$ et $R^3$ est sélectionné dans le groupe de radicaux d'hydrocarbure ayant de 1 à 70 atomes de carbone par radical, et où au moins un de $R^1$, $R^2$ et $R^3$ est sélectionné dans le groupe de radicaux d'hydrocarbure ayant de 10 à 80 atomes de carbone par radical.

5. Composition selon la revendication 4, où au moins un de $R^1$, $R^2$ et $R^3$ est sélectionné dans le groupe de radicaux d'hydrocarbure ayant de 1 à 40 atomes de carbone par radical, et où au moins un de $R^1$, $R^2$ et $R^3$ est sélectionné dans le groupe de radicaux d'hydrocarbure ayant de 40 à 80 atomes de carbone par radical.

6. Composition selon l'une quelconque des revendications précédentes, où ladite silicone est soluble dans du benzène.

7. Composition selon l'une quelconque des revendications précédentes, où ladite silicone a une viscosité dans la plage de 1 à 1 000 000 cSt.

8. Emulsion eau-dans-huile, émulsion huile-dans-eau ou émulsion non aqueuse comprenant la composition selon l'une quelconque des revendications précédentes.

9. Composition cosmétique comprenant une composition de silicone d'organosiloxane ramifiée comprenant un noyau en résine silicone, où ledit noyau en résine silicone comprend:

(a) deux ou plusieurs unités de siloxane de la formule structurelle (I):

$$SiO_{4/2} \qquad (I)$$

où lesdites unités de siloxane sont liées de manière covalente, soit directement les unes aux autres soit indirectement par un ou plusieurs groupes d'organosiloxane de pontage et
(b) un ou plusieurs groupes terminaux ayant la formule structurelle (II):

$$R^1{}_3SiO_{1/2} \qquad (II)$$

où lesdits groupes d'organosiloxane de pontage sont sélectionnés dans le groupe d'organosiloxanes ayant les formules structurelles (III) et (IV):

$$R^2{}_2SiO_{2/2} \qquad (III)$$

$$R^3SiO_{3/2} \qquad (IV)$$

où chaque $R^1$ dans chaque groupe terminal (II), chaque $R^2$ dans chaque groupe de pontage (III) et chaque $R^3$ dans chaque groupe de pontage (IV) est indépendamment un radical d'hydrocarbure,

**caractérisée en ce que**
chaque $R^1$ dans chaque groupe terminal (II), chaque $R^2$ dans chaque groupe de pontage (III) et chaque $R^3$ dans chaque groupe de pontage (IV) est indépendamment un radical d'hydrocarbure alicyclique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 21130600 P
- US 5493041 A
- US 4987169 A
- US 5760116 A
- EP 1164171 A
- EP 0958804 A
- EP 0694573 A
- EP 0827983 A
- EP 1164172 A
- US 6060546 A
- US 09033788 B

**Non-patent literature cited in the description**

- *U.S. Food and Drug Administration's,* 10 October 1993